Europäisches Patentamt

European Patent Office

Office européen des brevets

(11) Publication number: **0 403 048**
**A2**

## (12) EUROPEAN PATENT APPLICATION

(21) Application number: **90302203.6**

(22) Date of filing: **01.03.90**

(51) Int. Cl.5: **A61K 31/70**

The title of the invention has been amended (Guidelines for Examination in the EPO, A-III, 7.3).

(30) Priority: **14.06.89 US 366099**

(43) Date of publication of application:
**19.12.90 Bulletin 90/51**

(84) Designated Contracting States:
**BE DE DK ES FR GB GR IT NL**

(71) Applicant: **WARNER-LAMBERT COMPANY**
**201 Tabor Road**
**Morris Plains New Jersey 07950(US)**

(72) Inventor: **Granger, Colin D.**
**615 Hillside Road**
**Chester, New Jersey 07930(US)**
Inventor: **Barry, Eileen P.**
**20 Edsall Drive**
**Sussex, New Jersey 07461(US)**

(74) Representative: **Jones, Michael Raymond et al**
**HASELTINE LAKE & CO. Hazlitt House 28**
**Southampton Buildings Chancery Lane**
**London WC2A 1AT(GB)**

(54) Medicated compositions containing sucralfate and processes for their production.

(57) There are disclosed novel medicated compositions useful in treating a wide variety of disorders. The novel compositions comprise sucralfate and a therapeutically effective amount of a medicament. In a preferred embodiment, the medicament is selected from the group consisting of antacids, antiulcer medicaments, gastro-unstable medicaments, and medicaments which are normally administered in divided doses two or more times daily. Such medicated compositions are useful in treating various disorders by providing a mucous membrane adherent protective gel in the stomach having the medicament entrapped therein. The novel medicated compositions may be utilized in a wide variety of pharmaceutical products. This invention also relates to methods by which these medicated compositions and pharmaceutical products may be prepared.

EP 0 403 048 A2

# MEDICATED COMPOSITIONS AND PROCESSES FOR THEIR PRODUCTION

This invention pertains to novel medicated compositions useful in treating a wide variety of disorders, and to their preparation.

Peptic ulcers are lesions in the mucous membrane of the oesophagus, stomach or duodenum caused by gastric acid and pepsin. Peptic ulcers are believed to be caused by hypersecretion of gastric fluids or decreased resistance of the mucous membrane to the action of gastric acids and pepsin.

The methods for treating ulcers include diet modification, surgical removal of the ulcer, and the use of drugs. Useful antiulcer drugs include antacids, which neutralize excess acid secretion; anticholinergics, which diminish acid secretion; histamine $H_2$ receptor blocking agents, which block gastric acid secretion; prostaglandins, which increase mucous membrane resistance to gastric fluids; prokinetic agents, which enhance gastrointestinal motility; and gel forming compositions, which form ulcer protective barriers.

A gel forming drug in this last category is sucralfate, which is well known in the art as a basic aluminium sucrose sulphate complex which accelerates the healing of peptic ulcers. Sucralfate is described in The Merck Index, Tenth Edition, 1983, page 1273, and in Martindale, The Extra Pharmacopoeia, 27th Edition, published by The Pharmaceutical Press, London, 1977, page 1815. The mechanism by which sucralfate works is not fully understood but it is known that the sulfated disaccharide is not absorbed from the gastrointestinal tract and that the antiulcer activity is therefore exerted locally and not systemically. Sucralfate has a greater affinity for ulcerated mucosa than for non-ulcerated mucosa and produces morphological and functional changes in the mucosa. These changes include mucus release, changes in ion transport and increased release and synthesis of prostaglandins (i.e., prostaglandin $E_2$) from the mucosa.

At pH values below about 3.5, sucralfate coagulates to form a gel-like mass. In the gastrointestinal tract, this gel-like mass concentrates at ulcer sites to produce an ulcer adherent cytoprotective barrier with proteinaceous exudate. This protein barrier (a) binds to the ulcer site to form a protective barrier which blocks diffusion of hydrogen ions, (b) adsorbs bile salts and inhibits the potential ulcerogenic properties of pepsin, and (c) blocks back diffusion of gastric acid across the sucralfate protein barrier. The antiulcer effects of sucralfate are not attributed to neutralization of gastric acid because sucralfate has negligible acid-neutralizing capability.

The pH dependency of sucralfate gel formation presents drawbacks in administering sucralfate antiulcer therapy. Antacids, for example, which are also usually administered to subjects in need of antiulcer therapy, may not be concurrently administered with sucralfate. Antacids generally raise the pH value in the gastrointestinal tract to levels which inhibit sucralfate gel formation.

Furthermore, because of the complexity of ulcer disease, complete curing of peptic ulcers is rarely accomplished regardless of the particular drug employed. For example, growing evidence suggests that the microorganism Cambylobacter pylori causes duodenal ulcers to recur after apparently successful treatment, see C.D. Dooley et al., Annals of Internal Medicine: 108, pp. 70-79 (1988). Consequently, drug combinations, such as an antimicrobial agent and an antibiotic, have been used to mediate against ulcer recurrence. Suitable antibacterial bismuth preparations and antibiotics used to treat Campylobacter pylori are disclosed in United Kingdom patent applications no. 2,195,890 and 2,195,891, European patent applications no. A2 206,626, A2 206,625, A2 206,627, and A1 217,440, and PCT patent application no. WO86/05981.

United States patent no. 4,772,470, issued to Inoue et al. and assigned to Nitto Electric Industrial Co., Ltd., discloses an oral bandage comprising a film support for a soft adhesive film comprised of a mixture of a polycarboxylic acid and/or a polycarboxylic acid anhydride and a vinyl acetate polymer. The oral bandage may have incorporated therein a topical drug such as sucralfate for administration to the oral mucosa.

United States patent no. 4,704,278, issued to Wu et al. and assigned to American Home Products Corp., discloses an aqueous antacid composition of fluidized magaldrate suspension comprising magaldrate gel (aluminum hydroxide and magnesium hydroxide) and a fluidizing amount of a combination of an aluminum hydroxide gel as a first fluidizer and a pharmaceutically acceptable citric ion source as a second fluidizer. The composition may also contain other therapeutically active substances such as sucralfate.

United States patent no. 4,676,984, also issued to Wu et al. and assigned to American Home Products Corp., discloses an aqueous antacid composition of fluidized magaldrate suspension comprising magaldrate gel and a fluidizing amount of a combination of an aluminum hydroxide gel as a first fluidizer and a pharmaceutically acceptable citric ion source as a second fluidizer, and a polyhydric alcohol. The composition may also contain other therapeutically active substances such as sucralfate.

United States patent no. 4,670,185, issued to Fujiwara et al. and assigned to Lion Corporation, discloses an aqueous vesicle dispersion comprising (1) a nonionic surfactant selected from polyethylene castor oil ethers and polyethylene hydrogenated castor oil ethers, (2) a nonionic surfactant which is a sorbitan

2

polyester, and (3) an ionic surfactant. The dispersions may be used as release-controlled agents and may contain topical agents such as peptic ulcer remedy medicaments which include sucralfate.

United States patent no. 4,615,697, issued to Robinson and assigned to Bio-Mimetics, Inc., discloses a controlled release composition comprising a bioadhesive comprised of a water-swellable but water-insoluble carboxy-functional polymer and a treating agent which may be sucralfate.

M. Itch et al., Gastroenterology, 96, p. A229 (May 1989), "Combination of EGF and Sucralfate Significantly Accelerates The Healing of Chronic Gastric Ulcers In the Rat," discloses the combination of epidermal growth factor (EGF) and sucralfate for treatment of chronic gastric ulcers. Sucralfate is said to enable EGF to remain in the stomach at a high concentration.

While the above medicated compositions may provide some degree of improved cytoprotective activity and antiulcer therapy, none of the compositions has overcome the problem of antacid inhibition of sucralfate gel formation. Moreover, complete curing of peptic ulcers is rarely accomplished regardless of the particular drug employed. Protective gel barrier compositions alone do not kill microorganisms and antibacterial preparations do not heal ulcers, and are known to raise toxicity problems. Other treatments may render subjects hypochlorhydric (low hydrochloric acid levels) inviting other gastrointestinal disorders such as infections and carcinomas.

Thus is would be desirable to develop a cytoprotective formulation in which sucralfate and an antacid could be administered concurrently as well as a formulation in which antiulcer drugs could be administered in combination to produce an enhanced antiulcer effect.

According to one aspect of the present invention, there is provided a medicated composition which comprises sucralfate and a therapeutically effective amount of at least one of (a) a substantially water-insoluble medicament and (b) a mixture of a water-soluble medicament and a release delaying material, wherein the medicament is premixed with a release delaying material to form a substantially water-insoluble medicament.

According to another aspect of the present invention, there is provided a process for preparing a medicated composition, which comprises:

(a) admixing a therapeutically effective amount of a water-soluble medicament with a release delaying material to form a water-insoluble medicament; and

(b) admixing sucralfate with the mixture of step (a).

The present invention pertains to novel medicated compositions useful in treating a wide variety of disorders. The novel compositions comprise sucralfate and a therapeutically effective amount of a medicament.

In a preferred embodiment, the medicament is selected from the group consisting of antacids, antiulcer medicaments, gastro-unstable medicaments, and medicaments which are normally administered in divided doses two or more times daily. Such medicated compositions are useful in treating various disorders by providing a mucous membrane adherent protective gel in the stomach having the medicament entrapped therein. The novel medicated compositions may be utilized in a wide variety of pharmaceutical products. This invention also relates to methods by which these medicated compositions and pharmaceutical products may be prepared.

In one embodiment of the present invention, the novel medicated compositions are cytoprotective compositions which comprise sucralfate and a therapeutically effective amount of an antacid which is, or which may be made, substantially water-insoluble. Such cytoprotective compositions are useful in treating peptic ulcers by forming an ulcer adherent protective gel barrier in which the antacid is entrapped thereby providing localized and sustained neutralization of gastric acidity at the ulcer site.

In another embodiment of the present invention, the novel medicated compositions are cytoprotective compositions which comprise sucralfate and a therapeutically effective amount of an antiulcer medicament. These cytoprotective compositions are useful in treating peptic ulcers by forming an ulcer adherent protective gel barrier in which the antiulcer medicament is entrapped thereby providing localized and sustained antiulcer therapy at the ulcer site.

In still another embodiment of the invention, the novel medicated compositions are semi-enteric-protected compositions which comprise sucralfate and a therapeutically effective amount of a gastro-unstable medicament. These semi-enteric-protected compositions are useful in treating a wide variety of disorders by forming a mucous membrane adherent protective gel mass in the stomach in which the gastro-unstable medicament is entrapped and protected.

The term "enteric" means: of, relating to, or being a medicinal preparation treated to pass through the stomach unaltered and disintegrate in the intestines. The term "semi-enteric" as used in the specification and claims herein means: of, relating to, or being a medicinal preparation treated to not disintegrate in the stomach for a period of time sufficient to allow a sucralfate gel to form in the stomach.

In yet another embodiment of the invention, the novel medicated compositions are sustained release compositions which comprise sucralfate and a therapeutically effective amount of a medicament normally administered in divided doses two or more times daily. These sustained release compositions are useful in treating a wide variety of disorders by providing a protective mucous membrane adherent gel in the stomach in which the medicament is entrapped, thereby providing localized and sustained release of the medicament to the stomach walls in therapeutically effective amounts.

The medicaments which are useful in the present invention are substantially water-insoluble or which may be made substantially water-insoluble by admixing the medicament with a release delaying material. The medicament must be substantially insoluble in the gastric fluids since the sucralfate gel will not efficiently entrap a medicament in solution. In one embodiment, the medicament is substantially insoluble or practically insoluble in water. Because medicaments generally considered substantially insoluble, or practically insoluble, may nevertheless have some small yet finite solubility in water, applicant defines the substantially water-insoluble medicaments in the present invention to include medicaments which at therapeutic levels are sufficiently insoluble, or sufficiently slow to dissolve in, or react with, gastric fluids that (a) the medicament will be substantially entrapped in the sucralfate gel before it dissolves in the gastric fluids, and/or (b) the medicament will not raise the pH value of the gastric fluids above about 3.5 and thereby inhibit gel formation of sucralfate and prevent subsequent entrapment of the medicament in the gel. In general, these substantially water-insoluble medicaments are less than about 1% soluble in water.

In another embodiment of the present invention, the medicament is a water-soluble medicament which can be made substantially water-insoluble by combining the medicament with an encapsulating or release delaying material. The encapsulated or delayed release form employed in this invention may be any such form well known in the art, including water-based coatings, solvent-based coatings, colloidal (latex) and near colloidal (pseudolatex) aqueous dispersion coatings. Lipids may also be used to form a liposome-type material or lipo-medicament. By encapsulating the normally water-soluble medicament to form a release delaying or liposome-type material, the medicament is delayed or prevented from dissolving in, and/or raising the pH value of, the gastric fluids. The term "water-soluble medicament" as used herein also includes those medicaments which are considered substantially insoluble in gastric fluids but which nevertheless react with and neutralize gastric fluids, or quickly dissolve in gastric fluids, and must be combined with a release delaying material to ensure entrapment of the medicament in the gel and/or to prevent the medicament from raising the gastric pH value and thereby inhibit sucralfate gel formation.

In a preferred embodiment, the water-soluble medicament is combined with a lipid to form a substantially water-insoluble medicament. The term "liposome" means a structure composed of lipid bilayers that enclose a volume. The term "lipids," as used herein, means hydrophobic substances which are relatively insoluble in water but generally soluble in organic solvents and includes oils, fats and the like. The most commonly used lipid is phospholipid. The weight ratio of lipid to medicament in the liposome-type materials is that amount of lipid to medicament necessary to prevent the medicament from inhibiting sucralfate gel formation and/or that amount necessary to prevent the medicament from dissolving in the gastric fluids, while not significantly preventing the medicament from exerting its therapeutic activity. The weight ratio of lipid to medicament is subject to such factors as the nature of the particular lipid used, the nature of the particular medicament used, and the desired final product. The exact weight ratio of lipid to medicament may be varied in order to obtain the result desired in the final product and such variations are within the capabilities of those skilled in the art without the need for undue experimentation.

In general, liposomes are prepared by dispersing a lipid such as a phospholipid in an aqueous phase. A heterogeneous mixture of vesicular structures is usually formed most of which contain multiple lipid bilayers forming concentric spherical shells commonly known as multilamellar vesicles. Sonication of the dispersion converts the multilamellar vesicles to small unilamellar vesicles which contain only a single bilayer. Large unilamellar vesicles can be prepared by such well known techniques as infusion procedures, reverse-phase evaporation and detergent dilution. In an infusion procedure, a lipid solution in a nonpolar solvent is infused into an aqueous solution. In a reverse-phase evaporation procedure, a dispersion of inverted micells of lipid is produced in a system containing a mixed organic and aqueous phase. A variety of techniques have been used to dilute detergent originally codispersed with a lipid. The preparation of liposomes has been described by, for example, M. J. Ostro, Ed., Liposomes, Marcel Dekker, Inc., New York, pp. 27-53 (1983), which disclosure is incorporated herein by reference. The preparation of liposomes is well known in the art and is not the subject of the present invention.

A method is provided for localizing the neutralizing activity of an antacid at an ulcer site which comprises administering to a subject a cytoprotective composition comprising sucralfate and a therapeutically effective amount of an antacid which is, or which may be made, substantially insoluble in water to form an ulcer adherent cytoprotective gel barrier in which the antacid is entrapped thereby providing localized neutralization of gastric acidity at the ulcer site. A method is also provided for extending the residence time of an antacid in the stomach for at least four (4) hours which comprises administering to a subject the inventive cytoprotective composition comprising sucralfate and a therapeutically effective amount of an antacid to form an ulcer adherent cytoprotective gel barrier in which the antacid is entrapped thereby providing sustained neutralization of gastric acidity at the ulcer site. A method is further provided for treating peptic ulcers (duodenal and gastric ulcers), type-B gastritis and dyspepsia which consists of administering to a subject the inventive cytoprotective composition comprising sucralfate and a therapeutically effective amount of an antacid thereby providing localized and sustained neutralization of gastric acidity at the ulcer site. Because the sustained neutralizing activity of the antacid is localized at the ulcer site and not throughout the stomach, the cytoprotective composition can neutralize gastric acidity more efficiently. The cytoprotective composition can therefore be formulated with a smaller amount of antacid medicament, with a reduction in associated side effects without a concomitant decrease in therapeutic benefits.

A method is also provided for localizing the therapeutic activity of an antiulcer medicament at an ulcer site which comprises administering to a subject a cytoprotective composition comprising sucralfate and a therapeutically effective amount of an antiulcer medicament to form an ulcer adherent cytoprotective gel barrier in which the antiulcer medicament is entrapped thereby providing localized antiulcer therapy at the ulcer site. A method is also provided for extending the residence time of an antiulcer medicament in the stomach for at least four (4) hours which comprises administering the inventive cytoprotective composition to a subject to form an ulcer adherent cytoprotective gel barrier in which the antiulcer medicament is entrapped thereby providing sustained antiulcer therapy at the ulcer site. A method is further provided for treating peptic ulcers (duodenal and gastric ulcers), type-B gastritis and dyspepsia which consists of administering to a subject the inventive cytoprotective composition thereby providing localized and sustained antiulcer therapy at the ulcer site. Because the therapy of the antiulcer medicament is sustained and localized at the ulcer site, and not throughout the stomach, the inventive cytoprotective composition can treat ulcers more efficiently. The cytoprotective composition can therefore be formulated with a smaller amount of antiulcer medicament, with a reduction in associated side effects without a concomitant decrease in therapeutic benefits. For example, bismuth compounds in therapeutically effective amounts to treat ulcers can raise toxicity problems. By localizing the therapeutic activity of the bismuth compound at the ulcer site, a sufficiently high concentration of the bismuth compound can be maintained at the ulcer without raising the toxicity problem throughout the body.

Still another method is provided for administering medicaments, which are normally degraded in the gastric fluids of the stomach, in semi-enteric-protected form. The method consists of administering to a subject a semi-enteric-protected composition comprising sucralfate and a therapeutically effective amount of a gastro-unstable medicament to form a mucous membrane adherent protective gel in the stomach in which the medicament is entrapped and protected. A method is also provided for administering a gastro-unstable medicament in semi-enteric-protected form, wherein the medicament is released in immediate and sustained release form in the stomach, which comprises administering to a subject the inventive semi-enteric-protected composition. Because the gastro-unstable medicament is entrapped in the mucous membrane adherent protective sucralfate gel, the medicament is protected from degradation by the gastric fluids of the stomach but may be absorbed in immediate and sustained release form through the stomach walls. By permitting a gastro-unstable medicament to be absorbed in the stomach, the inventive semi-enteric-protected composition can provide a quicker onset of pharmacological activity of the medicament.

Yet another method is provided for administering a medicament, normally administered in divided doses two or more times daily, in sustained release form. The method consists of administering to a subject a sustained release composition comprising sucralfate and a therapeutically effective amount of a medicament to form a mucous membrane adherent gel in which the medicament is entrapped and slowly released in the gel. A method is also provided for administering a medicament in sustained release form, wherein the medicament is released in the stomach and duodenum, which comprises administering to a subject the inventive sustained release composition to form a mucous membrane adherent gel in which the medicament is entrapped in the gel and released to the stomach walls. By entrapping the medicament in the mucous membrane adherent gel, the sustained release composition can release the medicament more slowly and more uniformly in the stomach, and then again in the duodenum at higher pH values, than conventional sustained release compositions.

The present invention also includes a method for localizing the therapeutic activity of a medicament in the stomach which comprises administering to a subject a medicated composition comprising sucralfate and a therapeutically effective amount of a substantially water-insoluble medicament. In yet another embodiment, the present invention includes a method for extending the residence time of a medicament in the stomach for at least four (4) hours which comprises administering to a subject a medicated composition comprising sucralfate and a therapeutically effective amount of a substantially water-insoluble medicament.

In another embodiment, the present invention includes a method for localizing the therapeutic activity of a medicament in the stomach which comprises administering to a subject a medicated composition comprising sucralfate and a therapeutically effective amount of a mixture of a water-soluble medicament and a release delaying material, wherein the medicament is premixed with the release delaying material to form a substantially water-insoluble medicament. The present invention also includes a method for extending the residence time of a medicament in the stomach for at least four (4) hours which comprises administering to a subject a medicated composition comprising sucralfate and a therapeutically effective amount of a mixture of a water-soluble medicament and a release delaying material, wherein the medicament is premixed with the release delaying material to form a substantially water-insoluble medicament.

As set out above, sucralfate, also known as alpha-D-glucopyranoside, beta-D-fructofuranosyl-, octakis-(hydrogen sulfate), aluminum complex, is a basic aluminum sucrose sulfate complex which accelerates the healing of peptic ulcers. Sucralfate is a white amorphous powder which is soluble in dilute acid and base but is practically insoluble in water. At pH values below about 3.5, sucralfate coagulates to form a gel-like mass. Sucralfate is commercially available under the tradename CARAFATE, which is distributed by Marion Laboratories, Kansas City, Mo.

In one embodiment of the invention, the medicament in the medicated composition is an antacid. Antacids are substances which counteract or neutralize acidity in the stomach. The antacids which are useful in the present invention substantially insoluble or practically insoluble in water. Because antacid medicaments generally considered substantially insoluble, or practically insoluble, may nevertheless have some small yet finite solubility in water, applicant defines the substantially water-insoluble antacids in the present invention to include antacids which at therapeutic levels are sufficiently insoluble, or sufficiently slow to dissolve in or react with gastric fluids that they will not raise the pH value of the gastric fluids above about 3.5 and thereby inhibit gel formation of sucralfate and prevent subsequent entrapment of the antacid in the sucralfate gel. In general, these antacids are less than about 1% soluble in water.

Non-limiting examples of substantially water-insoluble antacids are bismuth subcarbonate $[(BiO)_2CO_3]$, bismuth nitrate $[Bi(NO)_3]$, magnesium trisilicate $[2MgO.3SiO_2.xH_2O]$, dihydroxy aluminum sodium carbonate $[NaCO_3.Al(OH)_2]$, and the like, and mixtures thereof. The preferred substantially insoluble antacids are selected from the group consisting of bismuth subcarbonate and magnesium trisilicate, and mixtures thereof.

In another embodiment of the present invention, the antacid is a water-soluble antacid which can be made substantially water-insoluble by combining the antacid with an encapsulating or release delaying material. As set out above, by encapsulating the normally water-soluble antacid to form a release delaying or liposome-type material, the antacid is prevented from dissolving in, and raising the pH value of, the gastric fluids, and thereby inhibiting sucralfate gel formation. The term "water-soluble antacid" as used herein also includes those antacids which are substantially insoluble in gastric fluids but which nevertheless react with and neutralize gastric fluids and must be in a delayed release form to prevent the inhibition of sucralfate gel formation. As set out above, the preparation of delayed release forms and liposomes is well known in the art and is not the subject of the present invention.

Non-limiting examples of water-soluble antacids, which may be made substantially water-insoluble, in the present invention are sodium bicarbonate, calcium carbonate, sodium citrate, aluminum hydroxide, magnesium hydroxide, and the like, and mixtures thereof. The preferred water-soluble antacids are selected from the group consisting of aluminum hydroxide, calcium carbonate, and mixtures thereof.

The antacid medicament of the present invention may be used in many distinct physical forms well known in the pharmaceutical art to provide an initial dosage of the medicament and/or a time-release form of the smedicament. Without being limited thereto, such physical forms include free forms and encapsulated forms, and mixtures thereof.

In accordance with this invention, an improved cytoprotective composition may be prepared by combining sucralfate and a therapeutically effective amount of an antacid which is, or which may be made, substantially water-insoluble. The exact amount of antacid employed will vary with the particular antacid selected. In general, the dosage level of the antacid employed in the present invention will be up to the usual dosage employed for that antacid. Such dosages are known to the skilled practitioner in the medical arts and are not a part of the present invention. In a preferred embodiment, the improved cytoprotective compositions comprise an antacid present in an amount from about 10% to about 75%, by weight of the cytoprotective composition. In a more preferred embodiment, the cytoprotective compositions comprise an antacid present in an amount from about 20% to about 65%, by weight of the cytoprotective composition. In a most preferred embodiment, the cytoprotective compositions comprise an antacid present in an amount from about 25% to about 50%, by weight of the cytoprotective composition. The water-soluble antacids, which can be made substantially water-insoluble by combining the antacid with a release delaying material, may be employed in essentially the same amounts as the substantially water-insoluble antacids.

The improved cytoprotective compositions of the present invention can be prepared by conventional mixing techniques. The instant compositions are prepared by admixing sucralfate and the substantially water-insoluble antacid. It is critical that the resultant mass of sucralfate and antacid be mixed thoroughly until uniform to ensure entrapment of the antacid in the sucralfate mass during gel formation in the stomach. In the compositions employing a water-soluble antacid, the antacid is first uniformly admixed with a release delaying material, then admixed with sucralfate.

While the invention is not to be limited to theoretical considerations, applicant believes that the ulcer adherent protective gel barrier formed by sucralfate coupled with the acid neutralizing properties of the antacid entrapped therein results in a superior localized and sustained neutralizing effect at the ulcer site. Because of its insolubility, or poor or slow solubility in the gastric fluids, the antacid does not inhibit sucralfate gel formation but does retain its protective acid neutralizing properties at the ulcer site. Because the sustained neutralizing activity of the antacid is localized at the ulcer site and not throughout the stomach, the cytoprotective composition may neutralize gastric acidity more efficiently. The cytoprotective composition may therefore be formulated with less antacid medicament with a reduction in associated side effects without a concomitant decrease in therapeutic benefits. Furthermore, the sustained neutralizing properties of the gel entrapped antacid results in a superior cytoprotective agent over a prolonged period of time. The improved cytoprotective properties of the instant composition, which combine the distinct and differing modes of action of the sucralfate protective gelling component and the acid neutralizing antacid component, are markedly greater than that of either component alone. This enhanced cytoprotective property is not found in conventional sucralfate or antacid formulations. Accordingly, disadvantages associated with the use of sucralfate or antacids are reduced or eliminated.

In another embodiment of the invention, the medicament in the medicated composition is an antiulcer medicament (drugs, medicaments, pharmaceuticals) which may be selected from a wide variety of compounds and their acid addition salts. Both organic and inorganic salts may be used provided the compound maintains its medicament value. Exemplary acid salts include hydrochloride, hydrobromide, orthophosphate, benzoate, maleate, tartrate, succinate, citrate, salicylate, sulfate, acetate, carbonate and subcarbonate.

The antiulcer medicament may be selected from a wide range of well known antiulcer therapeutic agents and mixtures of therapeutic agents. Nonlimiting illustrative categories and specific examples include:

(a) Antibacterials, including bismuth containing compounds, such as, without limitation, bismuth aluminate, bismuth subcitrate, bismuth subgalate, bismuth subsalicylate, and mixtures thereof; the sulfonamides; the nitrofurans, such as nitrofurazone, nitrofurantoin, and furozolidone; and miscellaneous antibacterials such as metronidazole, tinidazole, nimorazole, and benzoic acid;

(b) Antibiotics, including the aminoglycosides, such as gentamycin, neomycin, kanamycin, and streptomycin; the macrolides, such as erythromycin, clindamycin, and rifamycin; the penicillins, such as penicillin G, penicillin V, ampicillin and amoxicillin; the polypeptides, such as bacitracin and polymyxin; the tetracyclines, such as tetracycline, chlorotetracycline, oxytetracycline, and doxycycline; the cephalosporins, such as cephalexin and cephalothin; and miscellaneous antibiotics, such as chloramphenicol, and clidamycin;

(c) Wound healing substances, which increase the mucous membrane resistance to gastric fluids, such as glycyrrhiza, glycyrrhizic acid, prostaglandin $E_2$, and prostaglandin $F_2$; and those compounds known to stimulate prostaglandin formation such as compounds of aluminum, magnesium, and bismuth, including aluminum hydroxide, magnesium trisilicate and bismuth subcarbonate, and mixtures thereof;

(d) Antispasmodics and Anticholinergics, which diminish acid secretion, such as poldine, glycopyrrolate, propantheline, isopropanamide, tincture of belladonna, and pirenzapine;

(e) Histamine $H_2$ receptor blocking agents, which block gastric acid secretion, such as cimetidine, ranitidine, and famotidine;

(f) Coagulating agents, which are substances that promote the coagulation of blood, such as thrombin;

(g) Adsorbants, which cleave microorganisms such as Campylobacter pylori, including clays, such as bentonite and smectite; and

(h) Anticancer preparations (antineoplastics), including antibiotics, antimetabolites, cytotoxic agents, nitrogen mustard derivatives, steroids and combinations thereof.

In a preferred embodiment, the antiulcer medicament is selected from the group consisting of

7

antibacterial compounds such as bismuth preparations. In another preferred embodiment, the antiulcer medicament is selected from the group consisting of antibiotic compounds, such as penicillin, erythromycin, deoxycycline, amoxicillin, ampicillin, and mixtures thereof. In a more preferred embodiment, the antiulcer medicament is a combination of compounds selected from the group consisting of antibacterial compounds, such as bismuth preparations and metronidazole, tinidazole, and nitrofurantoin, and antibiotic compounds, such as penicillin, erythromycin, deoxycycline, amoxicillin, ampicillin, and mixtures thereof.

As set out above, in the case where an antiulcer medicament dissolves readily in the gastric fluids and may not be efficiently entrapped in the sucralfate gel, or in the case where an antiulcer medicament may raise the gastric pH value of the gastric fluids above about 3.5 and thereby inhibit gel formation of sucralfate, the antiulcer medicament may be made substantially water-insoluble by combining the antiulcer medicament with an encapsulating or release delaying material. By encapsulating the otherwise water-soluble antiulcer medicament to form a release delaying material or liposome-type material, the antiulcer medicament is prevented from dissolving in, and/or raising the pH value of, the gastric fluids. The term "water-soluble antiulcer medicament" as used herein also includes those antiulcer medicaments which are considered substantially insoluble in gastric fluids but which nevertheless react with and neutralize gastric fluids, or quickly dissolve in gastric fluids, and must be combined with a release delaying material to ensure entrapment of the medicament in the gel and/or to prevent the medicament from raising the pH value and thereby inhibit sucralfate gel formation. As set out above, the preparation of release delaying materials and liposomes is well known in the art and is not the subject of the present invention.

The antiulcer medicament of the present invention may be used in many distinct physical forms well known in the pharmaceutical art to provide an initial dosage of the medicament and/or a time-release form of the medicament. Without being limited thereto, such physical forms include free forms and encapsulated forms, and mixtures thereof.

In accordance with this invention, an improved cytoprotective composition may be prepared by combining sucralfate and an antiulcer medicament in therapeutically effective amounts. The exact amount of antiulcer medicament present in the cytoprotective composition will vary depending upon the particular antiulcer medicament employed. In general, the dosage level of the antiulcer medicament employed in the cytoprotective composition will be up to the usual therapeutic dosage employed for that antiulcer medicament. Such dosages are known to the skilled practitioner in the medical arts and are not a part of the present invention. In a preferred embodiment, the improved cytoprotective compositions comprise an antiulcer medicament present in an amount from about 0.05% to about 33%, by weight of the cytoprotective composition. In a more preferred embodiment, the cytoprotective compositions comprise an antiulcer medicament present in an amount from about 0.5% to about 25%, by weight of the cytoprotective composition. In a most preferred embodiment, the cytoprotective compositions comprise an antiulcer medicament present in an amount from about 1% to about 20%, by weight of the cytoprotective composition.

The improved cytoprotective compositions of the present invention can be prepared by conventional mixing techniques. The instant compositions are prepared by admixing sucralfate and the antiulcer medicament. It is critical that the resultant mass of sucralfate and antiulcer medicament be mixed thoroughly until uniform to ensure entrapment of the antiulcer medicament in the sucralfate mass during gel formation in the stomach. In the compositions employing an antiulcer medicament and a release delaying material, the antiulcer medicament is first uniformly admixed with the release delaying material, then admixed with sucralfate.

While the invention is not to be limited to theoretical considerations, applicant believes that the ulcer adherent protective gel barrier formed by sucralfate coupled with the therapeutic properties of the antiulcer medicament entrapped therein results in a superior localized and sustained antiulcer therapy effect at the ulcer site. Because the sustained therapeutic effects of the antiulcer medicament are localized at the ulcer site, and not throughout the stomach, the cytoprotective composition can treat ulcers more efficiently. The cytoprotective composition can therefore be formulated with a smaller amount of antiulcer medicament with a reduction in associated side effects without a concomitant decrease in therapeutic benefits. Furthermore, the sustained therapeutic properties of the gel entrapped antiulcer medicament results in a superior cytoprotective agent over a prolonged period of time. The improved cytoprotective properties of the instant composition, which combine the distinct and differing modes of action of the sucralfate protective gelling component and the antiulcer component, are markedly greater than that of either component alone. This enhanced cytoprotective property is not found in conventional sucralfate and other antiulcer formulations. Accordingly, disadvantages associated with the use of sucralfate or antiulcer medicaments are reduced or eliminated.

In another embodiment of the invention, the medicament in the medicated composition is a gastro-unstable medicament (drug, pharmaceutical). Gastro-unstable medicaments are compounds which are degraded in the gastric fluids of the stomach and may be selected from a wide variety of compounds and their acid addition salts. Both organic and inorganic salts may be used provided the compound maintains its medicament value. Exemplary acid salts include hydrochloride, hydrobromide, orthophosphate, benzoate, maleate, tartrate, succinate, citrate, salicylate, sulfate and acetate.

The gastro-unstable medicaments may be selected from a wide range of therapeutic agents and mixtures of therapeutic agents. Nonlimiting illustrative categories and specific examples include:

(a) Peptides, in general;

(b) Prostaglandins, both natural and synthetic, such as prostaglandin $E_2$, prostaglandin $F_2$, and their analogues:

(c) Antibiotics, such as erythromycin and quinoline; and

(d) Coagulating agents , such as thrombin.

In a preferred embodiment, the gastro-unstable medicament is erythromycin.

As set out above, in the case where a gastro-unstable medicament dissolves readily in the gastric fluids and may not be efficiently entrapped in the sucralfate gel, or in the case where a gastro-unstable medicament may rapidly raise the pH value of the gastric fluids above about 3.5 and thereby inhibit gel formation of sucralfate, the gastro-unstable medicament may be made substantially water-insoluble by combining the gastro-unstable medicament with an encapsulating or release delaying material. By encapsulating the otherwise water-soluble gastro-unstable medicament to form a release delaying material or liposome-type material, the gastro-unstable medicament is prevented from rapidly dissolving in, and/or raising the pH value of, the gastric fluids. The term "water-soluble gastro-unstable medicament" as used herein also includes those gastro-unstable medicaments which are considered substantially insoluble in gastric fluids but which nevertheless react with and neutralize gastric fluids, or quickly dissolve in gastric fluids, and must be combined with a release delaying material to ensure entrapment of the medicament in the gel and/or to prevent the medicament from raising the pH value and thereby inhibit sucralfate gel formation. As set out above, the preparation of release delaying materials and liposomes is well known in the art and is not the subject of the present invention.

The gastro-unstable medicaments of the present invention may be used in many distinct physical forms well known in the pharmaceutical art to provide an initial dosage of the medicament and/or a time-release form of the medicament. Without being limited thereto, such physical forms include free forms and encapsulated forms, and mixtures thereof.

In accordance with this invention, an improved semi-enteric-protected composition may be prepared by combining sucralfate and a gastro-unstable medicament in therapeutically effective amounts. The exact amount of gastro-unstable medicament present in the semi-enteric-protected composition will vary depending upon the particular gastro-unstable medicament employed. In general, the dosage level of the gastro-unstable medicament employed in the semi-enteric-protected composition will be up to the usual therapeutic dosage employed for that gastro-unstable medicament. Such dosages are known to the skilled practitioner in the medical arts and are not a part of the present invention. In a preferred embodiment, the improved semi-enteric-protected compositions comprise a gastro-unstable medicament present in an amount from about 0.05% to about 33%, by weight of the semi-enteric-protected composition. In a more preferred embodiment, the improved semi-enteric-protected compositions comprise a gastro-unstable medicament present in an amount from about 0.5% to about 25%, by weight of the semi-enteric-protected composition. In a most preferred embodiment, the improved semi-enteric-protected compositions comprise a gastro-unstable medicament present in an amount from about 1% to about 20%, by weight of the semi-enteric-protected composition.

The improved semi-enteric-protected compositions of the present invention can be prepared by conventional mixing techniques. The instant compositions are prepared by admixing sucralfate and a gastro-unstable medicament. It is critical that the resultant mass of sucralfate and gastro-unstable medicament be mixed thoroughly until uniform to ensure entrapment of the gastro-unstable medicament in the sucralfate mass during gel formation in the stomach. In the compositions employing a gastro-unstable medicament and a release delaying material, the gastro-unstable medicament is first uniformly admixed with the release delaying material, then admixed with sucralfate.

While the invention is not to be limited to theoretical considerations, applicant believes that the mucous membrane adherent protective gel formed by sucralfate in the stomach coupled with the therapeutic properties of the semi-enteric-protected gastro-unstable medicament entrapped therein results in a superior composition which also provides immediate and sustained release of the gastro-unstable medicament through the stomach walls. Because the gastro-unstable medicament is slowly leached out of the gel at the stomach wall and is absorbed through the stomach wall, the semi-enteric-protected composition can provide

9

a quicker onset of pharmacological activity. In addition, therapeutic levels of gastro-unstable medicaments are maintained at the stomach wall for an extended period of time. Unprotected gastro-unstable medicaments would quickly be destroyed by the gastric environment affording little or therapeutic effect.

In another embodiment of the invention, the medicament in the medicated composition is a medicament (drug, pharmaceutical) which would normally be administered to a subject in divided doses two or more times daily. These medicaments, which are often administered in a sustained release form, generally undergo significant presystemic metabolism or have a relatively short elimination half-life. The medicaments may be selected from a wide variety of compounds and their acid addition salts. Both organic and inorganic salts may be used provided the compound maintains its medicament value. Exemplary acid salts include hydrochloride, hydrobromide, orthophosphate, benzoate, maleate, tartrate, succinate, citrate, salicylate, sulfate and acetate.

The medicament may be selected from a wide range of well known therapeutic agents and mixtures of therapeutic agents. Nonlimiting illustrative categories and specific examples include:

(a) Antibiotics, including the aminoglycosides, such as gentamycin, neomycin, kanamycin, and streptomycin; the macrolides, such as erythromycin, clindamycin, and rifamycin; the penicillins, such as penicillin G, penicillin V, ampicillin and amoxicillin; the polypeptides, such as bacitracin and polymyxin; the tetracyclines, such as tetracycline, chlorotetracycline, oxytetracycline, and doxycycline; the cephalosporins, such as cephalexin and cephalothin; and miscellaneous antibiotics, such as chloramphenicol and clidamycin;

(b) Cardiovascular preparations, including angiotensin converting enzyme (ace) inhibitors, such as catopril and lisinopril; antianginal preparations, such as nitroglycerin, isosorbide dinitrate and isosorbide dinitrate; antiarrhythmics, such as, quinidine gluconate and procainamide hydrochloride; antihypertensives, such as hydrochlorothiazide and methyldopa; beta-andrenergic receptor blocking agents, such as propanolol and atenolol: and calcium channel blockers, such as verapamil, diltiazem, and nifedipine;

(c) Analgesics, such as acetylsalicylic acid, acetaminophen, ibuprofen, isoxicam, meclofenamic acid, naproxen, phenacetin, phenylbutazone, salicylamide, and sodium salicylate;

(d) Antihistamines, such as chlorpheniramine, brompheniramine, phenindamine tartrate, pyrilamine maleate, methapyrilene fumarate, doxylamine succinate, phenyltoloxamine citrate, diphenylhydramine hydrochloride, promethazine, terfenadine and triprolidine;

(e) Decongestants, such as ephedrine, pseudoephedrine, phenylephrine, phenylpropanolamine;

(f) Antitussives, such as dextromethorphan, dextromethorphan hydrobromide, noscapine, carbetapentane citrate and chlophedianol hydrochloride;

(g) Bronchial dilators, such as terbutaline sulfate;

(h) Anti-inflammatories, such as sulfasalazine, isoxicam, meclophenamic acid and naproxen;

(i) Non-absorbable antibacterials, such as sulfaguanidine;

(j) Anti-cholesterolemic and anti-lipid agents such as gemfibrozil and lovastatin;

(k) Appetite suppressants such as phenylpropanolamine hydrochloride and caffeine;

(l) Stimulants such as nicotine;

(m) Expectorants such as guaifenesin; and

(n) Nutritional supplements, including vitamins and minerals, such as niacin, pantothenic acid, vitamin $B_6$ thiamine hydrochloride, riboflavin, potassium iodide, potassium chloride, cupric sulfate and ferrous sulfate.

Medicaments may be used alone or in combination within their normal dosage ranges.

In a preferred embodiment, the medicament is selected from the group consisting of anti-inflammatories, nonabsorbable antibacterials, and mixtures thereof. In a more preferred embodiment, the medicament is selected from the group consisting of sulfasalazine, sulfaguanidine, and mixtures thereof.

As set out above, in the case where a medicament dissolves readily in the gastric fluids and may not be efficiently entrapped in the sucralfate gel, or in the case where a medicament may raise the pH value of the gastric fluids above about 3.5 and thereby inhibit gel formation of sucralfate, the medicament may be made substantially water-insoluble by combining the medicament with an encapsulating or release delaying material. By encapsulating the otherwise water-soluble medicament to form a release delaying material or liposome-type material, the medicament is prevented from dissolving in, and/or raising the pH value of, the gastric fluids. The term "water-soluble medicament" as used herein also includes those medicaments which are considered substantially insoluble in gastric fluids but which nevertheless react with and neutralize gastric fluids, or quickly dissolve in gastric fluids, and must be combined with a release delaying material to ensure entrapment of the medicament in the gel and/or to prevent the medicament from raising the gastric pH value and thereby inhibit sucralfate gel formation. As set out above, the preparation of release delaying materials and liposomes is well known in the art and is not the subject of the present invention.

10

The medicaments of the present invention may be used in many distinct physical forms well known in the pharmaceutical art to provide an initial dosage of the medicament and/or a time-release form of the medicament. Without being limited thereto, such physical forms include free forms and encapsulated forms, and mixtures thereof.

The term "delayed release" as used in the specification and claims herein means that release of the medicament is delayed or postponed for a certain period of time and then substantially all medicament is released. The period of time that the release of medicament is delayed corresponds to the time required for sucralfate to gel in typical gastric conditions. The term "sustained release" as used in the specification and claims herein means that release of the medicament is continuous over an extended period of time with more than about 90% of the medicament being released over the period of time, the period of time being generally from about one hour to about 24 hours, and preferably from about one hour to about 12 hours.

In accordance with this invention, an improved sustained release composition may be prepared by combining sucralfate and a medicament in therapeutically effective amounts. The exact amount of medicament present in the sustained release composition will vary depending upon the particular medicament employed. In general, the dosage level of the medicament employed in the sustained release composition will be up to the usual therapeutic dosage employed for that medicament. Such dosages are known to the skilled practitioner in the medical arts and are not a part of the present invention. In a preferred embodiment, the improved sustained release compositions comprise a medicament present in an amount from about 0.05% to about 33%, by weight of the sustained release composition. In a more preferred embodiment, the improved sustained release compositions comprise a medicament present in an amount from about 0.5% to about 25%, by weight of the sustained release composition. In a most preferred embodiment, the improved sustained release compositions comprise a medicament present in an amount from about 1% to about 20%, by weight of the sustained release composition.

The improved sustained release compositions of the present invention can be prepared by conventional mixing techniques. The instant compositions are prepared by admixing sucralfate and the medicament. It is critical that the resultant mass of sucralfate and medicament be mixed thoroughly until uniform to ensure entrapment of the medicament in the resulting sucralfate gel. In the compositions employing a medicament and a release delaying material, the medicament is first uniformly admixed with the release delaying material, then admixed with sucralfate.

While the invention is not to be limited to theoretical considerations, applicant believes that the mucous membrane adherent protective gel formed by sucralfate in the stomach coupled with the therapeutic properties of the medicament entrapped therein results in a superior sustained release composition which also provides immediate absorption of the medicament through the stomach walls and in the duodenum. The protective mucous membrane adherent sucralfate gel, while delaying release of the medicament in the gastric fluids of the stomach, permits the medicament to be absorbed through the stomach wall. When the sucralfate gel passes from the stomach to the duodenum, the change in pH value (from acid to alkaline) causes the gel to collapse and thereby release the medicament in the duodenum where it can be absorbed. Because the medicament can be absorbed through the stomach, a sustained release composition can provide a quicker onset of pharmacological activity of the medicament. Furthermore, the release of the gel entrapped medicament in the duodenum results in a superior sustained release agent over a prolonged period of time and insures timely absorption of the medicament. This enhanced sustained release property is not found in conventional sustained release formulations. Accordingly, disadvantages associated with the use of sustained release compounds are reduced or eliminated.

The cytoprotective (including antacid and antiulcer medicaments), semi-enteric-protected, and sustained release medicated compositions of the present invention once prepared may be stored for future use or may be formulated with conventional additives, such as pharmaceutically acceptable carriers and confectionery bulking agents, to prepare a wide variety of pharmaceutical compositions to suit particular applications. Such compositions may be in the form of a lozenge, tablet, toffee, nougat, suspension, chewy candy, capsule, and the like. The pharmaceutically acceptable carriers may be prepared from a wide range of materials. Without being limited thereto, such materials include diluents, binders and adhesives, lubricants, disintegrants, coloring agents, bulking agents, flavoring agents, sweetening agents and miscellaneous materials such as buffers and adsorbents in order to prepare a particular medicated composition.

The medicated compositions of the present invention may be incorporated into a pharmaceutical composition in therapeutically effective amounts. Naturally, the exact amount of medicament employed will vary with the particular medicament selected. In general, the dosage level of the medicament employed in a pharmaceutical composition in the present invention will be the usual dosage employed for that medicament. Such dosages are known to the skilled practitioner in the medical arts and are not a part of the present invention.

The present invention extends to methods of making the pharmaceutical compositions. In such a method, a composition is made by (a) admixing sucralfate and a therapeutically effective amount of a substantially water-insoluble medicament to form a medicated composition, and (b) admixing a pharmaceutically acceptable carrier to the medicated composition of step (a). In another embodiment, a composition is made by (a) admixing a therapeutically effective amount of a water-soluble medicament with a release delaying material, (b) admixing sucralfate to the mixture of step (a) to form a medicated composition, and (c) admixing a pharmaceutically acceptable carrier to the medicated composition of step (b). Other ingredients will usually be incorporated into the composition as dictated by the nature of the desired composition as well known by those having ordinary skill in the art. The ultimate pharmaceutical compositions are readily prepared using methods generally known in the pharmaceutical arts.

The preparation of confectionery formulations is historically well known and has changed little through the years. Confectionery items have been classified as either "hard" confectionery or "soft" confectionery. The medicated compositions of the present invention can be incorporated into confectionery compositions by admixing the inventive compositions into conventional hard and soft confections.

As used herein, the term confectionery material means a product containing a bulking agent selected from a wide variety of materials such as sugar, corn syrup, and in the case of sugarless bulking agents, sugar alcohols such as sorbitol and mannitol and mixtures thereof. Confectionery material may include such exemplary substances as lozenges, tablets, toffee, nougat, suspensions, chewy candy, and the like. The bulking agent is present in a quantity sufficient to bring the total amount of composition up to 100%.

Lozenges are flavored medicated dosage forms intended to be sucked and held in the mouth. Lozenges may be in the form of various shapes such as flat, circular, octagonal and biconvex forms. The lozenge bases are generally in two forms: hard, boiled candy lozenges and compressed tablet lozenges.

Hard boiled candy lozenges may be processed and formulated by conventional means. In general, a hard boiled candy lozenge has a base composed of a mixture of sugar and other carbohydrate bulking agents kept in an amorphous or glassy condition. This amorphous or glassy form is considered a solid syrup of sugars generally having from about 0.5% to about 1.5% moisture. Such materials normally contain up to about 92% corn syrup, up to about 55% sugar and from about 0.1% to about 5% water, by weight of the final composition. The syrup component is generally prepared from corn syrups high in fructose, but may include other materials. Further ingredients such as flavoring agents, sweetening agents, acidulants, coloring agents and the like may also be added.

Boiled candy lozenges may also be prepared from non-fermentable sugars such as sorbitol, mannitol, and hydrogenated corn syrup. Typical hydrogenated corn syrups are Lycasin, a commercially available product manufactured by Roquette Corporation, and Hystar, a commercially available product manufactured by Lonza, Inc. The candy lozenges may contain up to about 95% sorbitol, a mixture of sorbitol and mannitol in a ratio from about 9.5:0.5 up to about 7.5:2.5, and hydrogenated corn syrup up to about 55%, by weight of the solid syrup component.

Boiled candy lozenges may be routinely prepared by conventional methods such as those involving fire cookers, vacuum cookers, and scraped-surface cookers also referred to as high speed atmospheric cookers.

Fire cookers involve the traditional method of making a boiled candy lozenge base. In this method, the desired quantity of carbohydrate bulking agent is dissolved in water by heating the agent in a kettle until the bulking agent dissolves. Additional bulking agent may then be added and cooking continued until a final temperature of 145° to 156° C. is achieved. The batch is then cooled and worked as a plastic-like mass to incorporate additives such as flavoring agents, coloring agents and the like.

A high-speed atmospheric cooker uses a heat-exchanger surface which involves spreading a film of candy on a heat exchange surface, the candy is heated to 165° to 170° C. in a few minutes. The candy is then rapidly cooled to 100° to 120° C. and worked as a plastic-like mass enabling incorporation of the additives, such as flavoring agents, coloring agents and the like.

In vacuum cookers, the carbohydrate bulking agent is boiled to 125° to 132° C., vacuum is applied and additional water is boiled off without extra heating. When cooking is complete, the mass is a semi-solid and has a plastic-like consistency. At this point, flavoring agents, coloring agents, and other additives are admixed in the mass by routine mechanical mixing operations.

The optimum mixing required to uniformly mix the flavoring agents, coloring agents and other additives during conventional manufacturing of boiled candy lozenges is determined by the time needed to obtain a uniform distribution of the materials. Normally, mixing times of from 4 to 10 minutes have been found to be acceptable.

Once the boiled candy lozenge has been properly tempered, it may be cut into workable portions or formed into desired shapes. A variety of forming techniques may be utilized depending upon the shape and size of the final product desired. A general discussion of the composition and preparation of hard confections may be found in H.A. Lieberman, Pharmaceutical Dosage Forms: Tablets, Volume 1 (1980), Marcel Dekker, Inc., New York, N.Y. at pages 339 to 469, which disclosure is incorporated herein by reference.

The apparatus useful in accordance with the present invention comprises cooking and mixing apparatus well known in the confectionery manufacturing arts, and therefore the selection of the specific apparatus will be apparent to the artisan.

In contrast, compressed tablet confections contain particulate materials and are formed into structures under pressure. These confections generally contain sugars in amounts up to about 95%, by weight of the composition, and typical tablet excipients such as binders and lubricants as well as flavoring agents, coloring agents and the like.

In addition to hard confectionery materials, the lozenges of the present invention may be made of soft confectionery materials such as those contained in nougat. The preparation of soft confections, such as nougat, involves conventional methods, such as the combination of two primary components, namely (1) a high boiling syrup such as a corn syrup, hydrogenated starch hydrolysate or the like, and (2) a relatively light textured frappe, generally prepared from egg albumin, gelatin, vegetable proteins, such as soy derived compounds, sugarless milk derived compounds such as milk proteins, and mixtures thereof. The frappe is generally relatively light, and may, for example, range in density from about 0.5 to about 0.7 grams/cc.

The high boiling syrup, or "bob syrup" of the soft confectionery is relatively viscous and has a higher density than the frappe component, and frequently contains a substantial amount of carbohydrate bulking agent such as a hydrogenated starch hydrolysate. Conventionally, the final nougat composition is prepared by the addition of the "bob syrup" to the frappe under agitation, to form the basic nougat mixture. Further ingredients such as flavoring agents, additional carbohydrate bulking agent, coloring agents, preservatives, medicaments, mixtures thereof and the like may be added thereafter also under agitation. A general discussion of the composition and preparation of nougat confections may be found in B.W. Minifie, Chocolate, Cocoa and Confectionery: Science and Technology, 2nd edition, AVI Publishing Co., Inc., Westport, Conn. (1980), at pages 424-425, which disclosure is incorporated herein by reference.

The procedure for preparing the soft confectionery involves known procedures. In general, the frappe component is prepared first and thereafter the syrup component is slowly added under agitation at a temperature of at least about 65o C., and preferably at least about 100o C. The mixture of components is continued to be mixed to form a uniform mixture, after which the mixture is cooled to a temperature below 80o C., at which point, the flavoring agent may be added. The mixture is further mixed for an additional period until it is ready to be removed and formed into suitable confectionery shapes.

The medicated compositions may also be in the form of a pharmaceutical suspension. Pharmaceutical suspensions of this invention may be prepared by conventional methods long established in the art of pharmaceutical compounding. Suspensions may contain adjunct materials employed in formulating the suspensions of the art. The suspensions of the present invention can comprise:

(a) preservatives such as benzoic acid, sorbic acid, methyl paraben, propyl paraben and ethylene-diaminetetraacetic acid (EDTA). Preservatives are generally present in amounts up to about 1%, and preferably from about 0.05% to about 0.5%, by weight of the suspension;

(b) buffers such as citric acid-sodium citrate, phosphoric acid-sodium phosphate, and acetic acid-sodium acetate in amounts up to about 1%, and preferably from about 0.05% to about 0.5%, by weight of the suspension;

(c) suspending agents or thickeners such as cellulosics like methylcellulose, carrageenans like alginic acid and its derivatives, xanthan gums, gelatin, acacis, and microcrystalline cellulose in amounts up to about 20%, and preferably from about 1% to about 15%, by weight of the suspension;

(d) antifoaming agents such as dimethyl polysiloxane in amounts up to about 0.2%, and preferably from about 0.01% to about 0.1%, by weight of the suspension;

(e) sweetening agents such as those sweeteners well known in the art, including both natural and artificial sweeteners. Sweetening agents such as monosaccharides, disaccharides and polysaccharides such as xylose, ribose, glucose (dextrose) mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, glycyrrhizin, and sugar alcohols such as sorbitol, mannitol, maltitol, hydrogenated starch hydrolysates and mixtures thereof may be utilized in amounts up to about 60%, and preferably from about 20% to about 50%, by weight of the suspension. Water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid

13

form of saccharin, and the like may be utilized in amounts from about 0.001% to about 5%, by weight of the suspension;

(f) flavoring agents such as those flavors well known to the skilled artisan, such as natural and artificial flavors and mints, such as peppermint, menthol, citrus flavors such as orange and lemon, artificial vanilla, cinnamon, various fruit flavors, both individual and mixed and the like may be utilized in amounts from about 0.5% to about 5%, by weight of the suspension;

(g) coloring agents such as pigments which may be incorporated in amounts up to about 6%, by weight of the suspension. A preferred pigment, titanium dioxide, may be incorporated in amounts up to about 2%, and preferably less than about 1%, by weight of the suspension. The coloring agents may also include natural food colors and dyes suitable for food, drug and cosmetic applications. These coloring agents are known as F.D.& C. dyes and lakes. The materials acceptable for the foregoing uses are preferably water-soluble. Such dyes are generally present in amounts up to about 0.25%, and preferably from about 0.05% to about 0.2%, by weight of the suspension;

(h) decoloring agents (decolorants, decolorizing agents) such as sodium metabisulfite, ascorbic acid and the like may be incorporated into the suspension to prevent color changes due to aging. In general, decolorizing agents may be used in amounts up to about 0.25%, and preferably from about 0.05% to about 0.2%, by weight of the suspension; and

(i) solubilizing agents (solubilizers) such as alcohol, propylene glycol, polyethylene glycol, and the like may be used to solubilize the flavoring agents. In general, solubilizing agents may be used in amounts up to about 10%, and preferably from about 2% to about 5%, by weight of the suspension.

The pharmaceutical suspensions of the present invention may be prepared as follows:

(A) admix the thickener with water heated from about 40o C. to about 95o C., preferably from about 40o C. to about 70o C., to form a dispersion if the thickener is not water-soluble or a solution if the thickener is water-soluble;

(B) admix the sweetening agent with water to form a solution;

(C) admix the medicated composition with the thickener-water admixture to form a uniform thickener-medicated composition;

(D) combine the sweetening solution with the thickener-medicated composition and mix until uniform; and

(E) admix the optional adjunct materials such as coloring agents, flavoring agents, decoloring agents, solubilizing agents, antifoaming agents, buffers and additional water with the mixture of step (D) to form the suspension.

The medicated compositions of this invention may also be in chewable form. To achieve acceptable stability and quality as well as good taste and mouth feel in a chewable formulation several considerations are important. These considerations include the amount of active substance per tablet, the flavoring agent employed, the degree of compressibility of the tablet and the organoleptic properties of the composition.

Chewable medicated candy is prepared by procedures similar to those used to make soft confectionery. In a typical procedure, a boiled sugar-corn syrup blend is formed to which is added a frappe mixture. The boiled sugar-corn syrup blend may be prepared from sugar and corn syrup blended in parts by weight ratio of about 90:10 to about 10:90. The sugar-corn syrup blend is heated to temperatures above about 120° C. to remove water and to form a molten mass. The frappe is generally prepared from gelatin, egg albumin, milk proteins such as casein, and vegetable proteins such as soy protein, and the like, which is added to a gelatin solution and rapidly mixed at ambient temperature to form an aerated sponge like mass. The frappe is then added to the molten candy mass and mixed until homogeneous at temperatures between about 65° C. and about 120° C.

The medicated composition of the instant invention can then be added to the homogeneous mixture as the temperature is lowered to about 65°-95° C. whereupon additional ingredients can then be added such as flavoring agents and coloring agents. The formulation is further cooled and formed into pieces of desired dimensions.

A general discussion of the lozenge and chewable tablet forms of confectionery may be found in H.A. Lieberman and L. Lachman, Pharmaceutical Dosage Forms: Tablets, Volume 1, Marcel Dekker, Inc., New York, N.Y. at pages 289 to 466, which disclosure is incorporated herein by reference.

In accordance with this invention, therapeutically effective amounts of the medicated compositions of the present invention may be incorporated into the hard and soft confections. Naturally, the exact amount of medicated composition employed will vary with the particular medicated composition selected. In general, the dosage level of the medicated composition employed in a pharmaceutical composition will be the usual dosage employed for that medicament. The amount of medicated composition may be varied in order to obtain the result desired in the final confectionery product and such variations are within the capabilities of those skilled in the art without the need for undue experimentation and are not a part of the present invention.

In a preferred embodiment, the medicated composition will be present in the medicated hard and soft confections in percentages by weight in an amount from about 0.1% to about 50%. In a more preferred embodiment, the medicated compositions will be present in the medicated hard and soft confections in percentages by weight in an amount from about 0.5% to about 40%. In a most preferred embodiment, the medicated composition will be present in the medicated hard and soft confections in percentages by weight in an amount from about 1% to about 25%. The pharmaceutically acceptable carrier and optional additives are present in a quantity sufficient to bring the total amount of the medicated confection composition up to 100%.

The present invention extends to methods of making the improved medicated hard and soft confection compositions. The medicated compositions may be incorporated into an othervise conventional hard or soft confection composition using standard techniques and equipment known to those skilled in the art. The apparatus useful in accordance with the present invention comprises mixing and heating apparatus well known in the confectionery manufacturing arts, and therefore the selection of the specific apparatus will be apparent to the artisan.

In such a method, a composition is made by (a) admixing sucralfate and a therapeutically effective amount of a substantially water-insoluble medicament to form a medicated composition, and (b) admixing a confectionery bulking agent to the medicated composition of step (a). In another embodiment, a composition is made by (a) admixing a therapeutically effective amount of a water-soluble medicament with a release delaying material, (b) admixing sucralfate to the mixture of step (a) to form a medicated composition, and (c) admixing a confectionery bulking agent to the medicated composition of step (b). Other ingredients will usually be incorporated into the composition as dictated by the nature of the desired composition as well known by those having ordinary skill in the art. The ultimate confectionery compositions are readily prepared using methods generally known in the food technology and pharmaceutical arts. Thereafter the confectionery mixture may be formed into desirable confectionery shapes.

The medicated compositions may be formulated with conventional ingredients which offer a variety of textures to suit particular applications. Such ingredients may be in the form of hard and soft confections, tablets, toffee, nougat, chewy candy, capsules, and so forth, both sugar and sugarless. The acceptable ingredients may be selected from a wide range of materials. Without being limited thereto, such materials include diluents, binders and adhesives, lubricants, disintegrants, bulking agents, humectants and buffers and adsorbents. The preparation of such confection products is well known.

The medicated compositions according to this invention are administered to subjects one or more times daily in therapeutically effective amounts. Naturally, the exact amount of medicament employed will vary with the particular medicament selected. In general, the dosage level of the medicament employed in the present invention will be the usual dosage employed for that medicament.

The present invention is further illustrated by the following examples which are not intended to limit the effective scope of the claims. All parts and percentages in the examples and throughout the specification and claims are by weight of the final composition unless otherwise specified.

EXAMPLES 1-6

Comparative Runs 1-6

These examples demonstrate in vitro comparative gelling experiments employing compositions comprising combinations of sucralfate and a substantially water-insoluble antacid (bismuth subcarbonate) and sucralfate and a water-soluble antacid (calcium carbonate) having the compositions set out in Table 1.

TABLE 1

| EXAMPLE | INGREDIENT | AMOUNT (Mg) |
|---|---|---|
| 1 | Sucralfate | 200 |
| 2 | $(BiO)_2CO_3$ | 200 |
| 3 | Sucralfate | 200 |
| | $(BiO)_2CO_3$ | 200 |
| 4 | Sucralfate | 200 |
| | $(BiO)_2CO_3$ | 100 |
| 5 | Sucralfate | 200 |
| | $CaCO_3$ | 200 |
| 6 | Sucralfate | 200 |
| | $CaCO_3$ | 100 |

The sucralfate used in these experiments was supplied by Nippon Gohsei, Osaka, Japan. Each composition in the above examples was added to an aliquot of 15ml of 0.1N hydrochloric acid in a test tube (21mm x 110mm) and then the composition was mixed with the aqueous acid solution by placing the test tube in a Vortex-Genie at a speed of 3 for 10-15 seconds.

The composition in example 1 gelled in about 10 seconds leaving an essentially clear supernatant. The composition in example 2 did not gel and the suspension lasted for about one minute. The composition in example 3 gelled in about 25 seconds leaving a slightly cloudy supernatant. The composition in example 4 gelled in about 15 seconds leaving an almost clear supernatant. The composition in example 5 did not gel and the suspension lasted for about one minute. The composition in example 6 did not gel immediately but some gelation was observed after about one minute, however, little antacid appeared to be entrapped in the gel.

The sucralfate compositions in examples 3 and 4, which contained the substantially water-insoluble antacid barium subcarbonate, gelled satisfactorily, trapping the barium subcarbonate in the sucralfate gel.

EXAMPLES 7-13

Comparative Runs 7-13

These examples demonstrate in-vitro comparative antimicrobial activity experiments employing compositions comprising sucralfate, bismuth subcarbonate, benzoic acid and combinations of sucralfate and bismuth subcarbonate, and sucralfate and benzoic acid having the compositions set out in Table 2.

TABLE 2

| EXAMPLE | INGREDIENT | AMOUNT (Mg) |
|---|---|---|
| 7 | Sucralfate | 200 |
| 8 | (BiO)$_2$CO$_3$ | 200 |
| 9 | Sucralfate | 200 |
| | (BiO)$_2$CO$_3$ | 200 |
| 10 | Benzoic Acid | 250 |
| 11 | Sucralfate | 200 |
| | Benzoic Acid | 250 |
| 12 | 0.1N Hydrochloric Acid | 0.2ml |
| 13 | Control | |

A quantity of 10ml of BHI broth was inoculated with 0.1ml of Escherichia coli, A.T.T.C. accession number 8739. The culture was incubated at 37° C. for a period of 18-24 hours. Bacto Plate Count Agar (Difco) plates were prepared and a well was made in the center of each plate. A quantity of 0.4ml of the above culture was introduced into the well in each plate. The plates were allowed to stand on the benchtop for about 20 minutes.

The sucralfate used in these experiments was supplied by Nippon Gohsei, Osaka, Japan. The compositions in examples 7-11 were each added to an aliquot of 15ml of 0.1N hydrochloric acid in a sterile test tube (21mm x 110mm), and then the composition was mixed with the aqueous acid solution by placing the test tube in a Vortex-Genie at a speed of 3 for 10-15 seconds.

The compositions in examples 7, 9, and 12 gelled and an aliquot of the gel from each composition was added to a well in an agar plate. The compositions in examples 8 and 10 did not gel and an aliquot of 0.2ml of the insoluble material in each composition was added to a well in an agar plate.

The compositions in examples 12 and 13 were used as controls. In example 12, an aliquot of 0.2ml of 0.1N hydrochloric acid was added to a well in an agar plate. In example 13, no test sample was added to the well which was inoculated only with the organism.

The above agar plates were then incubated at 37° C. for a period of 24 hours. Zones of inhibition were then determined and the results are shown in Table 3.

TABLE 3

| EXAMPLE | INGREDIENT | ZONE OF INHIBITION(CM) |
|---|---|---|
| 7 | Sucralfate | Negative |
| 8 | (BiO)$_2$CO$_3$ | Negative |
| 9 | Sucralfate | Negative |
| | (BiO)$_2$CO$_3$ | |
| 10 | Benzoic Acid | 5.5 |
| 11 | Sucralfate | 2.5 |
| | Benzoic Acid | |
| 12 | 0.1N Hydrochloric Acid | Negative |
| 13 | Control | Negative |

The compositions in examples 10 and 11, which contained benzoic acid and the combination of benzoic acid and sucralfate, respectively, showed zones of inhibition against Escherichia coli, A.T.T.C. accession number 8739.

In vitro, the antibacterial benzoic acid was released from the sucralfate gel during the incubation period as evidenced by the zone of inhibition. Accordingly, sucralfate can function as a carrier of antimicrobial agents.

This experiment was repeated and the results were reproduced.

The invention being thus described, it will be obvious that the same may be varied in many ways. Such variations are not to be regarded as a departure from the spirit and scope of the invention and all such modifications are intended to be included within the scope of the following claims.

PREFERRED ASPECT OF THE INVENTION INCLUDE :

1. A medicated composition which comprises sucralfate and a therapeutically effective amount of a substantially water-insoluble medicament.

2. The medicated composition according to aspect 1, wherein the medicament is an antacid medicament.

3. The medicated composition according to aspect 2, wherein the antacid medicament is selected from the group consisting of bismuth subcarbonate, bismuth nitrate, magnesium trisilicate, dihydroxy aluminum sodium carbonate, and mixtures thereof.

4. The medicated composition according to aspect 3, wherein the antacid medicament is selected from the group consisting of bismuth subcarbonate, magnesium trisilicate, and mixtures thereof.

5. The medicated composition according to aspect 2, wherein the antacid medicament is present in an amount from about 10% to about 75%, by weight of the medicated composition.

6. The medicated composition according to aspect 5, wherein the antacid medicament is present in an amount from about 20% to about 65%, by weight of the medicated composition.

7. The medicated composition according to aspect 1, wherein the medicament is an antiulcer medicament.

8. The medicated composition according to aspect 7, wherein the antiulcer medicament is selected from the group consisting of antibacterials, antibiotics, wound healing substances, antispasmodics and anticholinergics, histamine $H_2$ receptor blocking agents, coagulating agents, adsorbants, anticancer preparations, and mixtures thereof.

9. The medicated composition according to aspect 8, wherein the antiulcer medicament is selected from the group of antibacterials consisting of bismuth aluminate, bismuth subcitrate, bismuth subgalate, bismuth subsalicylate, and mixtures thereof.

10. The medicated composition according to aspect 8, wherein the antiulcer medicament is selected from the group of antibiotics consisting of the aminoglycosides, such as gentamycin, neomycin, kanamycin, and streptomycin; the macrolides, such as erythromycin, clindamycin, and rifamycin; the penicillins, such as penicillin G, penicillin V, ampicillin and amoxicillin; the polypeptides, such as bacitracin and polymyxin; the tetracyclines, such as tetracycline, chlorotetracycline, oxytetracycline, and doxycycline; the cephalosporins, such as cephalexin and cephalothin; and antibiotics, such as chloramphenicol, clidamycin, and mixtures thereof.

11. The medicated composition according to aspect 7, wherein the antiulcer medicament is a combination of compounds selected from the group consisting of antibacterials, antibiotics, and mixtures thereof.

12. The medicated composition according to aspect 7, wherein the antiulcer medicament is present in an amount from about 0.05% to about 33%, by weight of the medicated composition.

13. The medicated composition according to aspect 12, wherein the antiulcer medicament is present in an amount from about 0.5% to about 25%, by weight of the medicated composition.

14. The medicated composition according to aspect 1, wherein the medicament is a gastro-unstable medicament.

15. The medicated composition according to aspect 14, wherein the gastro-unstable medicament is selected from the group consisting of peptides, prostaglandins, antibiotics, coagulating agents, and mixtures thereof.

16. The medicated composition according to aspect 15, wherein the gastro-unstable medicament is erythromycin.

17. The medicated composition according to aspect 14, wherein the gastro-unstable medicament is present in an amount from about 0.05% to about 33%, by weight of the medicated composition.

18. The medicated composition according to aspect 17, wherein the gastro-unstable medicament is present in an amount from about 0.5% to about 25%, by weight of the medicated composition.

19. The medicated composition according ro aspect 1, wherein the medicament is a medicament normally administered in divided doses two or more times daily.

20. The medicated composition according to aspect 19, wherein the medicament is selected from the group consisting of antibiotics, cardiovascular preparations, analgesics, antihistamines, decongestants, antitussives, bronchial dilators, anti-inflammatories, non-absorbable antibacterials, anti-cholesterolemic, appetite suppressants, stimulants, expectorants, nutritional supplements, and mixtures thereof.

21. The medicated composition according to aspect 20, wherein the medicament is selected from the group consisting of anti-inflammatories, non-absorbable antibacterials, and mixtures thereof.

22. The medicated composition according to aspect 19, wherein the medicament is present in an amount from about 0.05% to about 33%, by weight of the medicated composition.

23. The medicated composition according to aspect 22, wherein the medicament is present in an amount from about 0.5% to about 25%, by weight of the medicated composition.

24. A medicated composition which comprises sucralfate and a therapeutically effective amount of a mixture of a water-soluble medicament and a release delaying material, wherein the medicament is premixed with the release delaying material to form a substantially water-insoluble medicament.

25. The medicated composition according to aspect 24, wherein the medicament is an antacid medicament.

26. The medicated composition according to aspect 25, wherein the antacid medicament is selected from the group consisting of sodium bicarbonate, calcium carbonate, sodium citrate, aluminum hydroxide, magnesium hydroxide, and mixtures thereof.

27. The medicated composition according to aspect 26, wherein the antacid medicament is selected from the group consisting of aluminum hydroxide, calcium carbonate, and mixtures thereof.

28. The medicated composition according to aspect 25, wherein the antacid medicament is present in an amount from about 10% to about 75%, by weight of the medicated composition.

29. The medicated composition according to aspect 28, wherein the antacid medicament is present in an amount from about 20% to about 65%, by weight of the medicated composition.

30. The medicated composition according to aspect 24, wherein the medicament is an antiulcer medicament.

31. The medicated composition according to aspect 30, wherein the antiulcer medicament is selected from the group consisting of antibacterials, antibiotics, wound healing substances, antispasmodics and anticholinergics, histamine $H_2$ receptor blocking agents, coagulating agents, adsorbants, anticancer preparations, and mixtures thereof.

32. The medicated composition according to aspect 30, wherein the antiulcer medicament is a combination of compounds selected from the group consisting of antibacterials, antibiotics, and mixtures thereof.

33. The medicated composition according to aspect 30, wherein the antiulcer medicament is present in an amount from about 0.05% to about 33%, by weight of the medicated composition.

34. The medicated composition according to aspect 33, wherein the antiulcer medicament is present in an amount from about 0.5% to about 25%, by weight of the medicated composition.

35. The medicated composition according to aspect 24, wherein the medicament is a gastrounstable medicament.

36. The medicated composition according to aspect 35, wherein the gastro-unstable medicament is selected from the group consisting of peptides, prostaglandins, antibiotics, coagulating agents, and mixtures thereof.

37. The medicated composition according to aspect 36, wherein the gastro-unstable medicament is erythromycin.

38. The medicated composition according to aspect 35, wherein the gastro-unstable medicament is present in an amount from about 0.05% to about 33%, by weight of the medicated composition.

39. The medicated composition according to aspect 38, wherein the gastrounstable medicament is present in an amount from about 0.5% to about 25%, by weight of the medicated composition.

40. The medicated composition according to aspect 24, wherein the medicament is a medicament normally administered in divided doses two or more times daily.

41. The medicated composition according to aspect 40, wherein the medicament is selected from the group consisting of antibiotics, cardiovascular preparations, analgesics, antihistamines, decongestants, antitussives, bronchial dilators, anti-inflammatories, non-absorbable antibacterials, anti-cholesterolemic, appetite suppressants, stimulants, expectorants, nutritional supplements, and mixtures thereof.

42. The medicated composition according to aspect 41, wherein the medicament is selected from the group consisting of anti-inflammatories, non-absorbable antibacterials, and mixtures thereof.

43. The medicated composition according to aspect 40, wherein the medicament is present in an amount from about 0.05% to about 33%, by weight of the medicated composition.

44. The medicated composition according to aspect 43, wherein the medicament is present in an amount from about 0.5% to about 25%, by weight of the medicated composition.

45. A method for preparing a medicated composition which comprises admixing sucralfate and a therapeutically effective amount of a substantially water-insoluble medicament.

46. The method according to aspect 45, wherein the medicament is an antacid medicament.

47. The method according to aspect 45, wherein the medicament is an antiulcer medicament.

48. The method according to aspect 45, wherein the medicament is a gastro-unstable medicament.

49. The method according to aspect 45, wherein the medicament is a medicament normally administered in divided doses two or more times daily.

50. A method for preparing a medicated composition which comprises:

(a) admixing a therapeutically effective amount of a water-soluble medicament with a release delaying material; and

(b) admixing sucralfate to the mixture of step (a).

51. The method according to aspect 50, wherein the medicament is an antacid medicament.

52. The method according to aspect 50, wherein the medicament is an antiulcer medicament.

53. The method according to aspect 50, wherein the medicament is a gastro-unstable medicament.

54. The method according to aspect 50, wherein the medicament is a medicament normally administered in divided doses two or more times daily.

55. A method for localizing the therapeutic activity of a medicament in the stomach which comprises administering to a subject a medicated composition comprising sucralfate and a therapeutically effective amount of a substantially water-insoluble medicament.

56. The method according to aspect 55, wherein the medicament is an antacid medicament.

57. The method according to aspect 55, wherein the medicament is an antiulcer medicament.

58. The method according to aspect 55, wherein the medicament is a gastro-unstable medicament.

59. The method according to aspect 55, wherein the medicament is a medicament normally administered in divided doses two or more times daily.

60. A method for localizing the therapeutic activity of a medicament in the stomach which comprises administering to a subject a medicated composition comprising sucralfate and a therapeutically effective amount of a mixture of a water-soluble medicament and a release delaying material, wherein the medicament is premixed with the release delaying material to form a substantially water-insoluble medicament.

61. The method according to aspect 60, wherein the medicament is an antacid medicament.

62. The method according to aspect 60, wherein the medicament is an antiulcer medicament.

63. The method according to aspect 60, wherein the medicament is a gastro-unstable medicament.

64. The method according to aspect 60, wherein the medicament is a medicament normally administered in divided doses two or more times daily.

65. A method for extending the residence time of a medicament in the stomach for at least four (4) hours which comprises administering to a subject a medicated composition comprising sucralfate and a therapeutically effective amount of a substantially water-insoluble medicament.

66. The method according to aspect 65, wherein the medicament is an antacid medicament.

67. The method according to aspect 65, wherein the medicament is an antiulcer medicament.

68. The method according to aspect 65, wherein the medicament is a gastro-unstable medicament.

69. The method according to aspect 65, wherein the medicament is a medicament normally administered in divided doses two or more times daily.

70. A method for extending the residence time of a medicament in the stomach for at least four (4) hours which comprises administering to a subject a medicated composition comprising sucralfate and a therapeutically effective amount of a mixture of a water-soluble medicament and a release delaying material, wherein the medicament is premixed with the release delaying material to form a substantially water-insoluble medicament.

71. The method according to aspect 70, wherein the medicament is an antacid medicament.

72. The method according to aspect 70, wherein the medicament is an antiulcer medicament.

73. The method according to aspect 70, wherein the medicament is a gastro-unstable medicament.

74. The method according to aspect 70, wherein the medicament is a medicament normally administered in divided doses two or more times daily.

75. A method for treating peptic ulcers which comprises administering to a subject a medicated cytoprotective composition comprising sucralfate and a therapeutically effective amount of a substantially water-insoluble antacid medicament.

76. A method for treating peptic ulcers which comprises administering to a subject a medicated cytoprotective composition .comprising sucralfate and a therapeutically effective amount of a mixture of a water-soluble antacid medicament and a release delaying material, wherein the medicament is premixed with the release delaying material to form a substantially water-insoluble antacid medicament.

77. A method for treating peptic ulcers which comprises administering to a subject a medicated cytoprotective composition comprising sucralfate and a therapeutically effective amount of a substantially water-insoluble antiulcer medicament.

78. A method for treating peptic ulcers which comprises administering to a subject a medicated cytoprotective composition comprising sucralfate and a therapeutically effective amount of a mixture of a water-soluble antiulcer medicament and a release delaying material, wherein the medicament is premixed with the release delaying material to form a substantially water-insoluble antiulcer medicament.

79. A method for localizing the neutralizing activity of an antacid medicament at an ulcer site which comprises administering to a subject a medicated cytoprotective composition comprising sucralfate and a therapeutically effective amount of a substantially water-insoluble antacid medicament.

80. A method for localizing the neutralizing activity of an antacid medicament at an ulcer site which comprises administering to a subject a medicated cytoprotective composition comprising sucralfate and a therapeutically effective amount of a mixture of a water-soluble antacid medicament and a release delaying material, wherein the antacid medicament is premixed with the release delaying material to form a substantially water-insoluble antacid medicament.

81. A method for administering a substantially water-insoluble gastro-unstable medicament in a semi-enteric-protected form which comprises administering to a subject a semi-enteric-protected medicated composition comprising sucralfate and a therapeutically effective amount of the gastro-unstable medicament.

82. A method for administering a water-soluble gastro-unstable medicament in a semi-enteric-protected form which comprises administering to a subject a semi-enteric-protected medicated composition comprising sucralfate and a therapeutically effective amount of a mixture of a water-soluble gastro-unstable medicament and a release delaying material, wherein the medicament is premixed with the release delaying material to form a substantially water-insoluble gastro-unstable medicament.

83. A method for administering in sustained release form a substantially water-insoluble medicament, normally administered in divided doses two or more times daily, which comprises administering to a subject a sustained release composition comprising sucralfate and a therapeutically effective amount of the medicament.

84. A method for administering in sustained release form a water-soluble medicament, normally administered in divided doses two or more times daily, which comprises administering to a subject a sustained release composition comprising sucralfate and a therapeutically effective amount of a mixture of the water-soluble medicament and a release delaying material, wherein the medicament is premixed with the release delaying material to form a substantially water-insoluble medicament.

85. A pharmaceutical composition which comprises a pharmaceutically acceptable carrier and a medicated composition wherein the medicated composition comprises sucralfate and a therapeutically effective amount of a substantially water-insoluble medicament.

86. The pharmaceutical composition according to aspect 85, wherein the medicament is an antacid medicament.

87. The pharmaceutical composition according to aspect 86, wherein the antacid medicament is selected from the group consisting of bismuth subcarbonate, bismuth nitrate, magnesium trisilicate, dihydroxy aluminum sodium carbonate, and mixtures thereof.

88. The pharmaceutical composition according to aspect 86, wherein the antacid medicament is present in an amount from about 10% to about 75%, by weight of the medicated composition.

89. The pharmaceutical composition according to aspect 85, wherein the medicament is an antiulcer medicament.

90. The pharmaceutical composition according to aspect 89, wherein the antiulcer medicament is selected from the group consisting of antibacterials, antibiotics, wound healing substances, antispasmodics and anticholinergics, histamine $H_2$ receptor blocking agents, coagulating agents, adsorbants, anticancer preparations, and mixtures thereof.

91. The pharmaceutical composition according to aspect 89, wherein the antiulcer medicament is present in an amount from about 0.05% to about 33%, by weight of the medicated composition.

92. The pharmaceutical composition according to aspect 85, wherein the medicament is a gastro-unstable medicament.

93. The pharmaceutical composition according to aspect 92, wherein the gastro-unstable medicament is selected from the group consisting of peptides, prostaglandins, antibiotics, coagulating agents, and mixtures thereof.

94. The pharmaceutical composition according to aspect 92, wherein the gastro-unstable medicament is present in an amount from about 0.05% to about 33%, by weight of the medicated composition.

95. The pharmaceutical composition according to aspect 85, wherein the medicament is a medicament normally administered in divided doses two or more times daily.

96. The pharmaceutical composition according to aspect 95, wherein the medicament is selected from the group consisting of antibiotics, cardiovascular preparations, analgesics, antihistamines, decongestants, antitussives, bronchial dilators, anti-inflammatories, non-absorbable antibacterials, anticholesterolemic, appetite suppressants, stimulants, expectorants, nutritional supplements, and mixtures thereof.

97. The pharmaceutical composition according to aspect 95, wherein the medicament is present in an amount from about 0.05% to about 33%, by weight of the medicated composition.

98. The pharmaceutical composition according to aspect 85, wherein the pharmaceutically acceptable carrier is selected from the group consisting of a hard confectionery, a soft confectionery, a lozenge, a tablet, a capsule, a toffee, a nougat, a suspension, and a chewy candy.

99. A pharmaceutical composition which comprises a pharmaceutically acceptable carrier and a medicated composition wherein the medicated composition comprises sucralfate and a therapeutically effective amount of a mixture of a water-soluble medicament and a release delaying material, wherein the medicament is premixed with the release delaying material to form a substantially water-insoluble medicament.

· 100. The pharmaceutical composition according to aspect 99, wherein the medicament is an antacid medicament.

101. The pharmaceutical composition according to aspect 100, wherein the antacid medicament is selected from the group consisting of sodium bicarbonate, calcium carbonate, sodium citrate, aluminum hydroxide, magnesium hydroxide, and mixtures thereof.

102. The pharmaceutical composition according to aspect 100, wherein the antacid medicament is present in an amount from about 10% to about 75%, by weight of the medicated composition.

103. The pharmaceutical composition according to aspect 99, wherein the medicament is an antiulcer medicament.

104. The pharmaceutical composition according to aspect 103, wherein the antiulcer medicament is selected from the group consisting of antibacterials, antibiotics, wound healing substances, antispasmodics and anticholinergics, histamine $H_2$ receptor blocking agents, coagulating agents, adsorbants, anticancer preparations, and mixtures thereof.

105. The pharmaceutical composition according to ash 103, wherein the antiulcer medicament is present in an amount from about 0.05% to about 33%, by weight of the medicated composition.

106. The pharmaceutical composition according to aspect 99, wherein the medicament is a gastro-unstable medicament.

107. The pharmaceutical composition according to aspect 106, wherein the gastro-unstable medicament is selected from the group consisting of peptides, prostaglandins, antibiotics, coagulating agents, and mixtures thereof.

108. The pharmaceutical composition according to aspect 106, wherein the gastro-unstable medicament is present in an amount from about 0.05% to about 33%, by weight of the medicated composition.

109. The pharmaceutical composition according to aspect 99, wherein the medicament is a medicament normally administered in divided doses two or more times daily.

110. The pharmaceutical composition according to aspect 109, wherein the medicament is selected from the group consisting of antibiotics, cardiovascular preparations, analgesics, antihistamines, decongestants, antitussives, bronchial dilators, anti-inflammatories, non-absorbable antibacterials, anticholesterolemic, appetite suppressants, stimulants, expectorants, nutritional supplements, and mixtures thereof.

111. The pharmaceutical composition according to aspect 109, wherein the medicament is present in an amount from about 0.05% to about 33%, by weight of the medicated composition.

. 112. The pharmaceutical composition according to aspect 99, wherein the pharmaceutically acceptable carrier is selected from the group consisting of a hard confectionery, a soft confectionery, a lozenge, a tablet, a capsule, a toffee, a nougat, a suspension, and a chewy candy.

113. A method for preparing a pharmaceutical composition which comprises:

(a) admixing sucralfate and a therapeutically effective amount of a substantially water-insoluble

22

medicament to form a medicated composition; and

(b) admixing a pharmaceutically acceptable carrier to the medicated composition of step (a).

114. A method for preparing a pharmaceutical composition which comprises:

(a) admixing a therapeutically effective amount of a water-soluble medicament with a release delaying material;

(b) admixing sucralfate to the mixture of step (a) to form a medicated composition; and

(c) admixing a pharmaceutically acceptable carrier to the medicated composition of step (b).


**Claims**

1. A medicated composition which comprises sucralfate and a therapeutically effective amount of at least one of (a) a substantially water-insoluble medicament and (b) a mixture of a water-soluble medicament and a release delaying material, wherein the medicament is premixed with a release delaying material to form a substantially water-insoluble medicament.

2. A medicated composition according to claim 1, wherein the medicament is an antacid medicament.

3. A medicated composition according to claim 2, wherein the antacid medicament is water-insoluble and is selected from bismuth subcarbonate, bismuth nitrate, magnesium trisilicate, dihydroxy aluminium sodium carbonate, and mixtures thereof, preferably bismuth subcarbonate, magnesium trisilicate, and mixtures thereof; or is water-soluble and is selected from sodium bicarbonate, calcium carbonate, sodium citrate, aluminium hydroxide, magnesium hydroxide, and mixtures thereof, preferably aluminium hydroxide, calcium carbonate, and mixtures thereof.

4. A medicated composition according to claim 2 or 3, wherein the antacid medicament is present in an amount from 10% to 75%, by weight of the medicated composition.

5. A medicated composition according to claim 4, wherein the antacid medicament is present in an amount from 20% to 65%, by weight of the medicated composition.

6. A medicated composition according to claim 1, wherein the medicament is an antiulcer medicament.

7. A medicated composition according to claim 6, wherein the antiulcer medicament is selected from antibacterials, antibiotics, wound healing substances, antispasmodics and anticholinergics, histamine $H_2$ receptor blocking agents, coagulating agents, adsorbants, anticancer preparations, and mixtures thereof.

8. A medicated composition according to claim 7, wherein the antiulcer medicament is an antibacterial selected from bismuth aluminate, bismuth subcitrate, bismuth subgalate, bismuth subsalicylate, and mixtures thereof.

9. A medicated composition according to claim 7, wherein the antiulcer medicament is an antibiotic selected from the aminoglycosides, preferably gentamycin, neomycin, kanamycin, and streptomycin; the macrolides, preferably erythromycin, clindamycin, and rifamycin; and penicillins, preferably penicillin G, penicillin V, ampicillin and amoxycillin; and polypeptides, preferably bacitracin and polymyxin; the tetracyclines, preferably tetracycline, chlorotetracycline, oxytetracycline, and doxycycline; the cephalosporins, preferably cephalexin and cephalothin; and antibiotics, preferably chloramphenicol and clidamycin; and mixtures thereof.

10. A medicated composition according to claim 1, wherein the medicament is a gastro-unstable medicament.

11. A medicated composition according to claim 10, wherein the gastro-unstable medicament is selected from peptides, prostaglandins, antibiotics, coagulating agents, and mixtures thereof.

12. A medicated composition according to claim 11, wherein the gastrounstable medicament is erythromycin.

13. A medicated composition according to claim 1, wherein the medicament is selected from antibiotics, cardiovascular preparations, analgesics, antihistamines, decongestants, antitussives, bronchial dilators, anti-inflammatories, non-absorbable antibacterials, anti-cholesterolemic, appetite suppressants, stimulants, expectorants, nutritional supplements, and mixtures thereof.

14. A medicated composition according to claim 13, where the medicament is selected from anti-inflammatories, non-absorbable antibacterials, and mixtures thereof.

15. A medicated composition according to any one of claims 6 to 14, wherein the medicament is present in an amount from 0.05% to 33%, by weight of the medicated composition.

16. A medicated composition according to claim 15, wherein the medicament is present in an amount from 0.5% to 25%, by weight of the medicated composition.

17. A pharmaceutical composition which comprises a pharmaceutically acceptable carrier and a medicated composition according to any preceding claim.

18. A pharmaceutical composition according to claim 17, wherein the pharmaceutically acceptable carrier is selected from a hard confectionery, a soft confectionery, a lozenge, a tablet, a capsule, a toffee, a nougat, a suspension, and a chewy candy.

19. A process for preparing a medicated composition, which comprises admixing sucralfate and a therapeutically effective amount of a substantially water-insoluble medicament.

20. A process for preparing a medicated composition, which comprises:

(a) admixing a therapeutically effective amount of a water-soluble medicament with a release delaying material to form a water-insoluble medicament; and

(b) admixing sucralfate with the mixture of step (a).

21. A process for preparing a pharmaceutical composition, which comprises:

(a) admixing sucralfate and therapeutically effective amount of a substantially water-insoluble medicament to form a medicated composition; and

(b) admixing a pharmaceutically acceptable carrier with the medicated composition of step (a).

22. A process for preparing a pharmaceutical composition, which comprises:

(a) admixing a therapeutically effective amount of a water-soluble medicament with a release delaying material to form a water-insoluble medicament;

(b) admixing sucralfate with the mixture of step (a) to form a medicated composition; and

(c) admixing a pharmaceutically acceptable carrier with the medicated composition of step (b).

Claims for the following Contracting States: GR, ES

1. A process for producing a medicated composition which comprises sucralfate and a therapeutically effective amount of at least one of (a) a substantially water-insoluble medicament and (b) a mixture of a water-soluble medicament and a release delaying material, wherein the medicament is premixed with a release delaying material to form a substantially water-insoluble medicament, which process comprises admixing sucralfate and a therapeutically effective amount of a substantially water-insoluble medicament; wherein optionally the water-insoluble medicament is preformed by admixing a therapeutically effective amount of a water-soluble medicament with a release delaying material to form the water-insoluble medicament.

2. A process according to claim 1, wherein the medicament is an antacid medicament.

3. A process according to claim 2, wherein the antacid medicament is water-insoluble and is selected from bismuth subcarbonate, bismuth nitrate, magnesium trisilicate, dihydroxy aluminium sodium carbonate, and mixtures thereof, preferably bismuth subcarbonate, magnesium trisilicate, and mixtures thereof; or is water-soluble and is selected from sodium bicarbonate, calcium carbonate, sodium citrate, aluminium hydroxide, magnesium hydroxide, and mixtures thereof, preferably aluminium hydroxide, calcium carbonate, and mixtures thereof.

4. A process according to claim 2 or 3, wherein the antacid medicament is present in an amount from 10% to 75%, by weight of the medicated composition.

5. A process according to claim 4, wherein the antacid medicament is present in an amount from 20% to 65%, by weight of the medicated composition.

6. A process according to claim 1, wherein the medicament is an antiulcer medicament.

7. A process according to claim 6, wherein the antiulcer medicament is selected from antibacterials, antibiotics, wound healing substances, antispasmodics and anticholinergics, histamine $H_2$ receptor blocking agents, coagulating agents, adsorbants, anticancer preparations, and mixtures thereof.

8. A process according to claim 7, wherein the antiulcer medicament is an antibacterial selected from bismuth aluminate, bismuth subcitrate, bismuth subgalate, bismuth subsalicylate, and mixtures thereof.

9. A process according to claim 7, wherein the antiulcer medicament is an antibiotic selected from the aminoglycosides, preferably gentamycin, neomycin, kanamycin, and streptomycin; the macrolides, preferably erythromycin, clindamycin, and rifamycin; and penicillins, preferably penicillin G, penicillin V, ampicillin and amoxycillin; and polypeptides, preferably bacitracin and polymyxin; the tetracyclines, preferably tetracycline, chlorotetracycline, oxytetracycline, and doxycycline; the cephalosporins, preferably cephalexin and cephalothin; and antibiotics, preferably chloramphenicol and clidamycin; and mixtures thereof.

10. A process according to claim 1, wherein the medicament is a gastro-unstable medicament.

11. A process according to claim 10, wherein the gastro-unstable medicament is selected from peptides, prostaglandins, antibiotics, coagulating agents, and mixtures thereof.

12. A process according to claim 11, wherein the gastro-unstable medicament is erythromycin.

13. A process according to claim 1, wherein the medicament is selected from antibiotics, cardiovascular preparations, analgesics, antihistamines, decongestants, antitussives, bronchial dilators, anti-inflammatories, non-absorbable antibacterials, anti-cholesterolemic, appetite suppressants, stimulants, expectorants, nutritional supplements, and mixtures thereof.

14. A process according to claim 13, where the medicament is selected from anti-inflammatories, non-absorbable antibacterials, and mixtures thereof.

15. A process according to any one of claims 6 to 14, wherein the medicament is present in an amount from 0.05% to 33%, by weight of the medicated composition.

16. A process according to claim 15, wherein the medicament is present in an amount from 0.5% to 25%, by weight of the medicated composition.

17. A process for producing pharmaceutical composition, which comprises admixing a pharmaceutically acceptable carrier and a medicated composition produced according to any preceding claim.

18. A process according to claim 17, wherein the pharmaceutically acceptable carrier is selected from a hard confectionery, a soft confectionery, a lozenge, a tablet, a capsule, a toffee, a nougat, a suspension, and a chewy candy.

19. A process for preparing a medicated composition, which comprises admixing sucralfate and a therapeutically effective amount of a substantially water-insoluble medicament.

20. A process for preparing a medicated composition, which comprises:

(a) admixing a therapeutically effective amount of a water-soluble medicament with a release delaying material to form a water-insoluble medicament; and

(b) admixing sucralfate with the mixture of step (a).

21. A process for preparing a pharmaceutical composition, which comprises:

(a) admixing sucralfate and therapeutically effective amount of a substantially water-insoluble medicament to form a medicated composition; and

(b) admixing a pharmaceutically acceptable carrier with the medicated composition of step (a).

22. A process for preparing a pharmaceutical composition, which comprises:

(a) admixing a therapeutically effective amount of a water-soluble medicament with a release delaying material to form a water-insoluble medicament;

(b) admixing sucralfate with the mixture of step (a) to form a medicated composition; and

(c) admixing a pharmaceutically acceptable carrier with the medicated composition of step (b).